# EUROPÄISCHE PATENTANMELDUNG

(11) **EP 2 740 718 A1**
(43) Veröffentlichungstag der Anmeldung: **11.06.2014**
(21) Anmeldenummer: 12008097.3
(22) Anmeldetag: 04.12.2012
(51) Int. Cl.: C07C 1/24, C07C 11/02, C07C 11/04, C07C 11/06, C07C 11/08, C07C 11/10

(54) **Verfahren zur katalytischen Dehydratisierung von Olefinen**

(71) Anmelder: Linde Aktiengesellschaft, 80331 München (DE)
(72) Erfinder: Ponceau, Marianne, 81371 München (DE); Rehak, Petra, 01277 Dresden (DE); Schulte, Sonja, 82515 Wolfratshausen (DE); Winkler, Florian, 80339 München (DE)

(57) **Zusammenfassung**

Die Erfindung betrifft ein Verfahren zur Herstellung von Olefinen durch katalytische Dehydratisierung von Alkohol oder Alkoholgemischen, bei welchem ein Eduktstrom (E), welcher ein Wasser-Alkohol-Gemisch aus einem oder mehreren Alkohole und Wasser enthält,

(a) auf einen Druck zwischen 25 bar und 80 bar verdichtet wird,

(b) der verdichtete Eduktstrom (E) bei einer Temperatur zwischen 250 °C und 600 °C verdampft und überhitzt wird,

(c) der verdichtete und überhitzte Eduktstrom (E) in einen ersten, isothermen Reaktor (4) geführt wird, in welchem der Eduktstrom (E) durch katalytische Dehydratisierung zu einem Zwischenproduktstrom (Z) umgesetzt wird und

(d) der Zwischenproduktstrom (Z) in mindestens einen weiteren Reaktor (6) geführt wird, in welchem der Zwischenproduktstrom (Z) durch katalytische Dehydratisierung umgesetzt wird, so dass ein olefinhaltiger Produktstrom (P) erhalten wird.

Das erfindungsgemäße Verfahren wird insbesondere zur Herstellung von Ethylen und Propylen verwendet.

## Beschreibung

Die Erfindung betrifft ein Verfahren zur Herstellung von Olefinen durch katalytische Dehydratisierung von Alkohol oder Alkoholgemischen.

Die Dehydratisierung (engl. dehydration) von Alkoholen zu Olefinen durch Abspaltung von Wasser in Gegenwart eines Katalysators ist eine bekannte Reaktion. So kann beispielsweise durch Dehydratisierung von Ethanol Ethylen (auch als Ethen bezeichnet) oder durch Dehydratisierung von Propanol Propylen (auch als Propen bezeichnet) hergestellt werden. Bei der Dehydratisierung handelt es sich um eine stark endotherme Reaktion.

Die WO 2009/098268 und die WO 20091098267 beinhalten eine Dehydratisierung von Alkohol in einem adiabatischen Reaktor unter Zugabe einer inerten Komponente wie Ethan, Kohlenwasserstoffen mit 3 bis 10 Kohlenstoffen, Naphthenen oder Kohlendioxid. In der WO 2011/037681 ist eine Dehydratisierung eines Alkohols in der flüssigen Phase bei hohem Druck beschrieben. In GB 2094829 ist eine Dehydratisierung von Ethanol in einem adiabatischen Reaktor bei hohem Druck. Die JP 1034929 beinhaltet eine Dehydratisierung von Ethanol in einem isothermen Reaktor, welchem zur Erhöhung der Konversion ein adiabatischer Reaktor nachgeschaltet ist. Die WO 20071/34415 betrifft die Rückführung von Wasser bei der Herstellung von Ethylen aus Ethylalkohol in einem adiabatischen Reaktor. Die US 4232179 beschreibt eine Dehydratisierung von Ethylalkohol in einem adiabatischen Reaktor bei hohen Temperaturen mit indirektem Wärmeübertrag mittels eines Wärmeüberträgerfluids.

Der Erfindung liegt die Aufgabe zugrunde, ein wirtschaftliches Verfahren zur Herstellung von Olefinen durch Dehydratisierung von Alkoholen anzugeben. Insbesondere soll mit der Erfindung ein energetisch vorteilhaftes Verfahren angegeben werden.

Diese Aufgabe wird durch ein Verfahren, das folgende Verfahrensschritte umfasst, gelöst:
- ein Eduktstrom, welcher ein Wasser-Alkohol-Gemisch aus einem oder mehreren Alkoholen und Wasser enthält, auf einen Druck zwischen 25 bar und 80 bar verdichtet wird,
- der verdichtete Eduktstrom verdampft auf eine Temperatur zwischen 250 °C und 600 °C und überhitzt wird,
- der verdichtete und überhitzte Eduktstrom in einen ersten, isothermen Reaktor geführt wird, in welchem der Eduktstrom durch katalytischen Dehydratisierung zu einem Zwischenproduktstrom umgesetzt wird,
- der Zwischenproduktstrom in mindestens einen weiteren Reaktor geführt wird, in welchem der Zwischenproduktstrom durch katalytische Dehydratisierung umgesetzt wird, so dass ein olefinhaltiger Produktstrom erhalten wird.

Als wesentlicher Vorteil des erfindungsgemäßen Verfahrens zeigt sich, dass sich durch das synergetische Zusammenwirken der Merkmale der Erfindung eine große Energieeinsparung im Vergleich zu den bekannten und bisher üblichen Verfahren ergibt. Da der Eduktstrom vor dem Verdampfen und Überhitzen verdichtet wird, ist der Energieverlust am Verdampfer, Überhitzer und isothermen Reaktor aufgrund des besseren Wärmeübergangs deutlich niedriger als ohne vorherige Verdichtung des Eduktstroms. Darüber hinaus ist keine Zugabe einer inerten Komponente, wie beispielsweise Dampf, welche in vielen Vergleichsprozessen für einen ausreichenden Wärmeeintrag in den Reaktor sorgt, notwendig. Bei dem erfindungsgemäßen Verfahren genügt als Wärmeträger vielmehr der Eduktstrom selbst. Dazu muss es sich aber bei dem an erster Stelle stehenden Reaktor um einen isothermen Reaktor handeln, der eine niedrigere Reaktionstemperatur aufweisen kann als wie in dem oder den weiteren Reaktoren benötigt wird und welcher den Eduktstrom lediglich In einen Zwischenproduktstrom umsetzt. Ein solcher Reaktor kann, zumal dort hoher Druck herrscht, mit geringerem Energieaufwand isotherm gehalten werden. Zudem kann der isotherme Reaktor auch klein dimensioniert sein, da ja weitere Reaktoren, in welchen der Zwischenproduktstrom weiter umgesetzt wird, folgen, so dass schließlich der olefinhaltige Produktstrom erhalten wird. In dem isothermen Reaktor wird das Katalysatormaterial für die endothermen Reaktionen im gesamten Behältervolumen genutzt und die notwendige Reaktionsenergie stets zugeführt. Es ist energetisch und konstruktiv sehr aufwändig einen Reaktor für endotherme Reaktionen isotherm zu gestalten. Auch sind für einen isothermen Reaktor im Vergleich zu einem adiabatischen Reaktor höhere Investitionskosten zu veranschlagen. Es ist deshalb sehr von Vorteil, dass der isotherme Reaktor im diesen Verfahren möglichst klein dimensioniert werden kann und somit die Investitionskosten des isothermen Reaktors als absolute Größe im Rahmen bleibt. Zu der möglichen, verhältnismäßig kleinen Dimensionierung des isothermen Reaktors trägt auch wesentlich der hohe Druck von 25 bis 80 bar bei, auf welchen der Eduktstrom verdichtet wird, da aufgrund der Verdichtung des Eduktstroms das notwendige Reaktorvolumen klein gehalten werden kann.

Da in dem erfindungsgemäßen Verfahren für den Eduktstrom ein Wasser-Alkohol-Gemisch verwendet werden kann, ermöglicht die Erfindung eine Verwendung von Alkoholen, welche aufgrund ihrer Herstellung einen hohen Wassergehalt aufweisen. So entstehen bei der Fermentation oder Gärung von Zuckern aus Biomasse Wasser-Alkohol-Gemische mit hohem Wassergehalt. Der Wassergehalt muss dem Wasser-Alkohol-Gemisch durch Destillation entzogen werden. Je reiner der Alkohol dabei werden soll, desto aufwändiger und energieverbrauchender ist die Destillation. Insbesondere eine Reinheit über das azeotrope Gemisch hinaus ist mit erheblichem Aufwand verbunden und kann nur beispielsweise durch Adsorption oder Dampfpermeation erreicht werden. Mit dem erfindungsgemäßen Verfahren ist es nun möglich, Wasser-Alkohol-Gemische zu verwenden, welche bei ihrer Herstellung nur eine einfache Destillation durchliefen. Aus diesem Grund führt das erfindungsgemäße Verfahren, insbesondere wenn die Herstellung des Alkohols mit einbezogen wird, insgesamt zu erheblichen Energieeinsparungen.

Diese eben genannten Vorteile zeigen sich besonders ausgeprägt, wenn ein Wasser-Alkohol-Gemisch verwendet wird, welches zwischen 20 Vol.-% und 90 Gew.-% bevorzugt zwischen 30 Gew.-% und 80 Gew.-%, weiter bevorzugt zwischen 40 Gew.-% und 70 Gew.-%, besonders bevorzugt zwischen 50 Gew.-% und 60 Gew.-% Wasser enthält.

In vorteilhafter Ausgestaltung der Erfindung wird zumindest der letzte Reaktor, vorzugsweise der zweite und alle auf den zweiten folgenden Reaktoren, adiabatisch betrieben. Adiabatische Reaktoren sind für die Umsetzung des Zwischenproduktstroms In den olefinhaltigen Produktstrom von Vorteil. Zudem sind diese weniger aufwändig in Bezug auf Konstruktion und Betrieb als isotherme Reaktoren. Auch ist es energetisch von Vorteil adiabatische Reaktoren zu verwenden, auch wenn dann eine Energiezufuhr zwischen den adiabatischen Reaktoren erfolgen muss.

Mit besonderen Vorteilen liegt die Temperatur des Zwischenproduktstroms beim Eintritt in den zweiten, vorzugsweise adiabatischen, Reaktor um mindestens 10°C, vorzugsweise um mindestens 20°C über der Temperatur des ersten, isothermen Reaktors. Der erste, isotherme Reaktor kann somit bei niedrigerer Temperatur betrieben werden, wie als Ausgangstemperatur für die Reaktion in dem zweiten, vorzugsweise adiabatischen, Reaktor notwendig ist. Dies ist möglich, da in dem ersten, isothermen Reaktor nicht eine vollständige, sondern lediglich eine Umsetzung in einen Zwischenproduktstrom erfolgt und in dem oder den weiteren Reaktoren erst die weitere Umsetzung durch katalytische Dehydratisierung in die Olefine erfolgt Von Vorteil ist dies, da dadurch eine Pyrolyse des Stoffstroms und die daraus resultierende Verkokung des Reaktors vermindert wird. Reaktoren für die Deyhdratisierung von Alkoholen verkoken sehr leicht und ihre Reinigung ist aufwändig. Die Verkokung ist umso geringer, je niedriger die Temperatur im Reaktor. Deshalb ist es von Vorteil, dass mit dem erfindungsgemäßen Verfahren der isotherme Reaktor bei möglichst niedrigen Temperaturen betrieben werden kann. In dieser vorteilhaften Ausgestaltung der Erfindung wird der Zwischenproduktstrom erwärmt, bevor er in den zweiten Reaktor geführt wird. Es kann jedoch in einer alternativen Ausgestaltung der Erfindung von Vorteil sein, den Zwischenproduktstrom ohne Erwärmung in den zweiten Reaktor zu führen. In diesem Fall liegt die Temperatur des Zwischenproduktstroms beim Eintritt in den zweiten, vorzugsweise adiabatisch betriebenen, Reaktor bei der Temperatur des isothermen Reaktors beziehungsweise wegen Verlusten etwas darunter. Eine Erwärmung findet dann vor einem Eintritt in den dritten Reaktor statt, welcher ebenfalls bevorzugt adiabatisch betrieben wird.

Mit besonderen Vorteilen sind im Zwischenproduktstrom Zwischenprodukte enthalten, welche in dem oder den weiteren Reaktoren größtenteils in Olefine umgesetzt werden. Der Zwischenproduktstrom enthält neben Wasser Oxygenate sowie Olefine und nicht umgesetzten Alkohol. Auch kann er Spuren von anderen Kohlenwasserstoffen und anderen Verbindungen enthalten. Aufgrund der Umsetzung der Zwischenprodukte in dem oder den weiteren Reaktoren in Olefine, kann der erste, isotherme Reaktor sehr klein ausgelegt werden und es zeigen sich die vorgenannten Vorteile. Durch gezielte Modifikation des Temperaturunterschiedes zwischen den einzelnen Reaktoren kann der Gehalt an Zwischen- und Nebenprodukten im olefinhaltigen Produktstrom, wie er letztendlich erhalten wird, minimiert werden. Damit wird gezielt die Ausbeute und Selektivität der Dehydratisierung gesteigert. Die Modifikation des Temperaturunterschieds kann durch einfaches Ausprobieren bestimmt werden. Vorteilhafterweise finden sich folglich im Produktstrom nach dem letzten adiabatischen Reaktor keine Zwischenprodukte mehr.

Mit Vorteil werden weder dem Eduktstrom noch dem Zwischenproduktstrom eine inerte Wärmeträgerkomponente, wie beispielsweise Wasser, Dampf oder sich inert verhaltende Kohlenwasserstoffe, zugegeben. Durch das erfindungsgemäße Verfahren ist die Herstellung der Olefine derartig gestaltet, dass dies nicht notwendig ist. So bildet sich in dem erfindungsgemäßen Verfahren der inerte Wärmeträger und Pyrolyseinhibitor Dampf bereits im ersten, isothermen Reaktor.

Vorteilhafterweise handelt es sich bei dem Wasser-Alkohol-Gemisch um eine Mischung aus Wasser und Alkoholen, die zwischen 2 und 10 Kohlenstoffatome aufweisen, beispielsweise um ein Wasser-Ethanol- oder um Wasser-Propanol- oder um Wasser-Butanol- oder um Wasser-Pentanol oder um Wasser-Ethanol-Propanol- oder um Wasser-Ethanol-Butanol- oder um Wasser-Ethanol-Pentanol- oder um Wasser-Ethanol-Propanol-Butanol- oder um Wasser-Ethanol-Propanol-Pentanol- oder um Wasser-Ethanol-Butanol-Pentanol- oder um Wasser-Propanol-Butanol- oder um Wasser-Ethanol-Propanol-Butanol-Pentanol-Gemische.

In einer vorteilhaften Ausgestaltung der Erfindung stammt das Wasser-Alkohol-Gemisch aus einer Fermentation. Durch Fermentation von Zucker können verschiedene Alkohole als Wasser-Alkohol-Gemische erhalten werden. Vorzugsweise wird das bei der Fermentation sich bildende Wasser-Alkohol-Gemisch in nur in einer einzigen Destillationsstufe zu höheren Alkoholanteilen aufkonzentriert. Für solchermaßen hergestellte Wasser-Alkohol-Gemische sind die oben erläuterten Vorteile hinsichtlich der Energieeinsparung besonders ausgeprägt. Auch entfallen bei der Herstellung des Wasser-Alkohol-Gemisches Investitionskosten für Rektifikation und Absolutierung, wodurch die Endprodukte der Dehydratisierung, also die Olefine, zu einem günstigeren Preis auf den Markt gebracht werden können. Für die Erfindung können jedoch auch technisch hergestellte Alkohole verwendet werden, die entsprechend Wasser enthalten oder denen Wasser zugesetzt wurde.

Als Katalysatoren werden vorzugsweise anorganische keramische Katalysatoren, vorzugsweise Al₂O₃ oder Alumosilikate verwendet. Es können jedoch auch beliebig andere Katalysatoren verwendet werden.

Vorteilhafterweise wird der Eduktstrom auf einen Druck zwischen 25 bar und 80 bar, vorzugsweise zwischen 30 bar und 50 bar verdichtet. In diesen Bereichen ist der Druck einerseits genügend hoch, so dass sich die erfindungsgemäßen Vorteile einstellen, und andererseits handelt es sich um Werte, bei welchen die Anlage technisch gut beherrschbar ist und die Investitionskosten im Rahmen bleiben.

Aus dem Produktstrom werden die Olefine gewonnen. Die Abtrennung und Gewinnung der Olefine wird mittels Verfahrensschritten durchgeführt, die aus der Herstellung von Olefinen bekannt sind und dort üblicherweise eingesetzt werden.

Vorzugsweise wird mit dem erfindungsgemäßen Verfahren Ethylen, Propylen, Butylen oder Penten hergestellt. Weiterhin können mit dem erfindungsgemäßen Verfahren auch die anderen entsprechenden Alkene hergestellt werden.

Im Folgenden soll die Erfindung nun anhand von Figuren in besonders vorteilhaften Ausgestaltungen beschrieben werden.

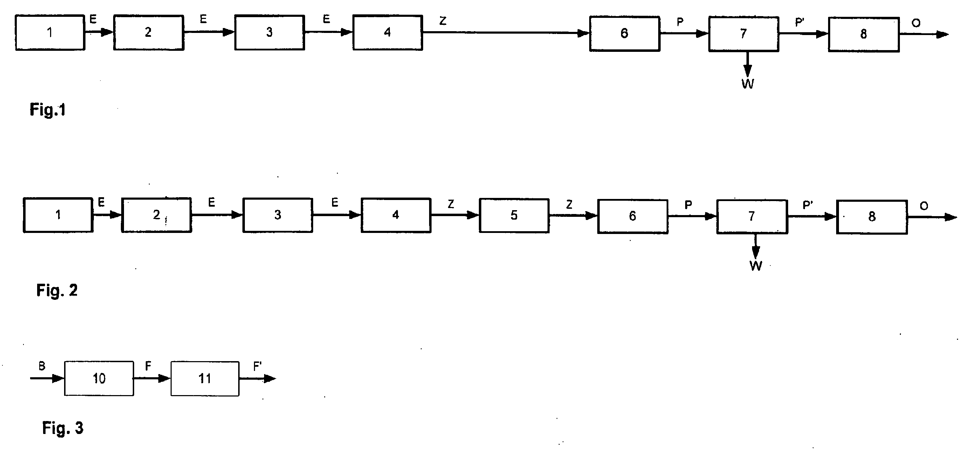

Figur 1 zeigt eine besonders vorteilhafte Ausgestaltung der Erfindung, die im Folgenden näher beschrieben wird: Aus einem Vorratsbehältnis 1 wird ein Wasser-Alkohol-Gemisch entnommen und als Eduktstrom E in einer Pumpstufe 2, welche vorzugsweise aus einer einzigen Pumpe besteht, auf eine Druck zwischen 25 und 80 bar verdichtet. Vorzugsweise umfasst die Pumpstufe 2 eine einzige Pumpe, jedoch kann auch eine mehrstufige Verdichtung bestehend aus mehreren Pumpen vorgesehen sein. Der verdichtete Eduktstrom E wird in einem Wärmeübertrager 3 verdampft und überhitzt. Der Wärmeübertrager 3 umfasst vorzugsweise zwei Wärmeübertrager, von welchen der erste den Eduktstrom E verdampft und der zweite den verdampften Eduktstrom E überhitzte. Der verdichtete und überhitzte Eduktstrom E wird nun in einen isothermen Reaktor 4 geführt, in welchem eine katalytische Dehydratisierung unter isothermen Bedingungen stattfindet. Dabei wird der Eduktstrom E in den Zwischenproduktstrom Z umgesetzt. Der Zwischenproduktstrom Z wird gemäß dieser Ausgestaltung der Erfindung direkt in den zweiten, vorzugsweise adiabatischen Reaktor 6 geführt. In diesem adiabatischen Reaktor 6 findet die weitere Umsetzung der katalytischen Dehydratisierung unter adiabatischen Bedingungen statt. Von Vorteil kann es sein, nicht nur einen adiabatischen, sondern zwei oder mehr adiabatische Reaktoren zu verwenden, in welchen die weitere katalytische Dehydratisierung erfolgt. Zwischen den adiabatischen Reaktoren ist eine Erwärmung des Stroms notwendig, damit das Temperaturniveau für die weitere katalytische Dehydratisierung in jedem der adiabatischen Reaktoren genügend hoch ist. In dem oder den adiabatischen Reaktor(en) 6 wird der Zwischenproduktstrom Z letztendlich in den olefinhaltigen Produktstrom P umgesetzt. Neben den gewünschten Olefinen enthält der Produktstrom P Wasser sowie geringe Verunreinigungen, welche insbesondere auf Nebenreaktionen zurückgehen. Aus dem Produktstrom P wird nun vorteilhafterweise zuerst das Wasser abgetrennt. Dazu wird der Produktstrom P in einen Stripper 7 geführt, in dem Wasser und Nebenprodukte abgetrennt werden und ein Produktstrom P' erhalten wird. Dieser restliche Produktstrom P' wird nun einer Reinigung 8, wie sie üblicherweise bei der Herstellung von Olefinen verwendet wird, zugeführt. Die Reinigung 8 kann dabei mehrere Stufen umfassen. Insbesondere umfasst die Reinigung 8 eine Tieftemperaturfraktionierung bei Drücken von ca. 20 bar. Vorteilhafterweise beträgt der Druck des Produktstromes deshalb nach dem Stripper 7 mindestens 20 bar. Eine Komprimierung des Produktstromes kann somit entfallen, wodurch die Kosten für Investition, Betrieb und Wartung für diese Komprimierung entfallen. Nach der Reinigung liegt ein Olefinstrom O vor, der aus dem gewünschten Olefin in der gewünschten Reinheit besteht.

Eine andere, ebenfalls besonders vorteilhafte Ausgestaltung wird nun mit Hilfe von Figur 2 beschrieben. Diese ist mit der mittels Figur 1 beschriebenen Ausgestaltung gleich; jedoch weist diese eine zusätzliche Erwärmung des Zwischenproduktstroms Z auf. Figur 2 zeigt deshalb alle Elemente der Figur 1 und zusätzlich ein Wärmeübertrager 5. Der Zwischenproduktstrom Z wird gemäß dieser Ausgestaltung zunächst in dieses Wärmeübertrager 5 geführt, bevor er in den adiabatischen Reaktor 6 geführt wird. Bei dem Wärmeübertrager 5 handelt es sich vorzugsweise um einen Wärmetauscher.

Wie bereits beschrieben, wird es mit dem erfindungsgemäßen Verfahren möglich, Wasser-Alkohol-Gemische, wie sie bei der Fermentation anfallen, als Edukt zu verwenden. Dies soll nun im Folgenden anhand der Ethanolherstellung und mit Hilfe von Figur 3 näher beschrieben werden. Biomasse B, wie beispielsweise Zucker, wird einem Fermentationsprozess 10 unterworfen, wobei ein Fermentationsprodukt F, im Beispiel Ethanol, entsteht, welches 6 bis 15 Gewichtsprozent Ethanol in Wasser enthält. Vorteilhafterweise wird das Fermentationsprodukt F einer Destillation 11 unterworfen, wodurch dem Wasser-Alkohol-Gemisch Wasser entzogen wird und ein destilliertes Fermentationsprodukt F' entsteht. Im Beispiel entsteht dabei ein Wasser-Ethanol-Gemisch mit 50 bis 90 Gewichtsprozent Wasser. Das destillierte Fermentationsprodukt F' kann nun als Eduktstrom E verwendet werden und der Pumpstufe 2 (siehe Figur 1 oder 2) zugeführt werden. Das destillierte Fermentationsprodukt F' kann zuvor auch in das Vorratsbehältnis 1 (siehe Figur 1 oder 2) geführt werden. Eine weitere Rektifikation im Azeotrop Wasser-Alkohol und insbesondere die aufwändige Trennung der Fermentationsnebenprodukte kann bei Einsatz des erfindungsgemäßen Verfahrens entfallen. Vielmehr kann der Fermentationsstrom F' mit einem hohen Wassergehalt der Dehydratisierung zugeführt werden. Investitionskosten, Betriebskosten und Wartung für die Rektifikation (ca. 95%) oder Absolutierung (100%) des Alkohols können so entfallen.

### Bezugszeichenliste

- 1: Vorratsbehältnis eines Wasser-Alkohol-Gemisches
- 2: Pumpstufe
- 3: Wärmeübertrager zum Verdampfen und Überhitzen
- 4: isothermer Reaktor
- 5: Wärmeübertrager
- 6: zweiter, vorzugsweiser adiabatischer Reaktor
- 7: Stripper
- 8: Reinigung

- 10: Fermentationsprozess
- 11: Destillation

- E: Eduktstrom
- Z: Zwischenproduktstrom
- P, P': Produktstrom
- O: Olefinstrom

- B: Biomasse
- F: Fermentationsprodukt
- F': destilliertes Fermentationsprodukt

## Patentansprüche

1. Verfahren zur Herstellung von Olefinen durch katalytische Dehydratisierung von Alkohol oder Alkoholgemischen, bei welchem
• ein Eduktstrom (E), welcher ein Wasser-Alkohol-Gemisch aus einem oder mehreren Alkoholen und Wasser enthält, auf einen Druck zwischen 25 bar und 80 bar verdichtet wird,
• der verdichtete Eduktstrom (E) verdampft auf eine Temperatur zwischen 250°C und 600°C und überhitzt wird,
• der verdichtete und überhitzte Eduktstrom (E) in einen ersten, isothermen Reaktor (4) geführt wird, in welchem der Eduktstrom (E) durch katalytische Dehydratisierung zu einem Zwischenproduktstrom (Z) umgesetzt wird,
• der Zwischenproduktstrom (Z) in mindestens einen weiteren Reaktor (6) geführt wird, in welchem der Zwischenproduktstrom (Z) durch katalytische Dehydratisierung umgesetzt wird, so dass ein olefinhaltiger Produktstrom (P) erhalten wird.

2. Verfahren nach Anspruch 1, **dadurch gekennzeichnet, dass** Wasser-Alkohol-Gemisch zwischen 20 Gew.-% und 90 Gew.-%, bevorzugt zwischen 30 Gew.-% und 80 Gew.-%, weiter bevorzugt zwischen 40 Gew.-% und 70 Gew.-%, besonders bevorzugt zwischen 50 Gew.-% und 60 Gew.-% Wasser enthält.

3. Verfahren nach Anspruch 1 oder 2, **dadurch gekennzeichnet, dass** zumindest der letzte Reaktor (6), vorzugsweise der zweite und alle auf den zweiten folgenden Reaktoren (6), adiabatisch betrieben werden.

4. Verfahren nach einem der Ansprüche 1 bis 3, **dadurch gekennzeichnet, dass** die Temperatur des Zwischenproduktstroms (Z) beim Eintritt in den zweiten Reaktor (6) um mindestens 10°C, vorzugsweise um mindestens 20°C über der Temperatur des ersten, isothermen Reaktors (4) liegt.

5. Verfahren nach einem der Ansprüche 1 bis 4, **dadurch gekennzeichnet, dass** im Zwischenproduktstrom (Z) Zwischenprodukte enthalten sind, welche in dem oder den weiteren Reaktoren (6) durch katalytischen Dehydratisierung in Olefine umgesetzt werden.

6. Verfahren nach einem der Ansprüche 1 bis 5, **dadurch gekennzeichnet, dass** weder dem Eduktstrom (E) noch dem Zwischenproduktstrom (Z) eine inerte Wärmeträgerkomponente zugegeben wird.

7. Verfahren nach einem der Ansprüche 1 bis 6, **dadurch gekennzeichnet, dass** es sich bei dem Wasser-Alkohol-Gemisch um eine Mischung aus Wasser und Alkoholen, die zwischen 2 und 10 Kohlenstoffatome aufweisen, bevorzugt um Wasser-Ethanol- oder um Wasser-Propanol- oder um Wasser-Butanol- oder Wasser-Pentanol-Gemische handelt.

8. Verfahren nach einem der Ansprüche 1 bis 7, **dadurch gekennzeichnet, dass** das Wasser-Alkohol-Gemisch aus einer Fermentation (10) stammen und nur in einer einzigen Destillationsstufe (11) zu höheren Alkoholanteilen aufkonzentriert wird.

9. Verfahren nach einem der Ansprüche 1 bis 8, **dadurch gekennzeichnet, dass** als Katalysator anorganische keramische Katalysatoren, vorzugsweise Al₂O₃ oder Alumosilikate verwendet werden.

10. Verfahren nach einem der Ansprüche 1 bis 9, **dadurch gekennzeichnet, dass** der Eduktstrom (E) auf einen Druck zwischen 25 bar und 80 bar, vorzugsweise zwischen 30 bar und 50 bar verdichtet wird.

11. Verfahren nach einem der Ansprüche 1 bis 10, **dadurch gekennzeichnet, dass** Ethylen, Propylen, Butylen oder Penten hergestellt wird.
